# FASCICULE DE BREVET EUROPEEN

(11) **EP 3 773 929 B1**
(45) Date de publication et mention de la délivrance du brevet: **08.06.2022**
(21) Numéro de dépôt: 19715955.1
(22) Date de dépôt: 11.04.2019
(51) Int. Cl.: A61K 8/97, A61Q 17/00, A61Q 19/00, A61Q 19/08, A61K 8/9783

(54) **PROCÉDÉ DE PRODUCTION D'UN EXTRAIT DE GRAINES D'ENTADA ENRICHI EN MÉTABOLITES D'INTÉRÊT, EXTRAIT OBTENU PAR UN TEL PROCÉDÉ ET APPLICATIONS COSMÉTIQUES ET DERMOCOSMETIQUES D'UN TEL EXTRAIT**
VERFAHREN ZUR HERSTELLUNG EINES MIT METABOLITEN VON INTERESSE ANGEREICHERTEN ENTADA-SAMEN-EXTRAKTES, DURCH SOLCH EIN VERFAHREN HERGESTELLTER EXTRAKT SOWIE KOSMETISCHE UND DERMOKOSMETISCHE ANWENDUNGEN SOLCH EINES EXTRAKTS
METHOD FOR PRODUCING AN ENTADA SEED EXTRACT ENRICHED WITH METABOLITES OF INTEREST, EXTRACT PRODUCED BY SUCH A METHOD, AND COSMETIC AND DERMOCOSMETIC APPLICATIONS OF SUCH AN EXTRACT

(30) Priorité: 12.04.2018 FR 1853216
(43) Date de publication de la demande: 17.02.2021
(73) Titulaire: Exsymol, 98000 Monaco (MC)
(72) Inventeur: SEGUIN, Marie-Christine, 98000 MONACO (MC)
(74) Mandataire: Cabinet Beau de Loménie
(86) Numéro de dépôt international: PCT/EP2019/059298
(87) Numéro de publication internationale: WO 2019/197548

(56) Documents cités:
- WO-A2-2014/165490
- CN-A- 106 031 498
- FR-A1- 2 832 418
- FR-A1- 2 874 503
- US-A- 3 641 243
- US-A1- 2009 098 617
- US-A1- 2011 129 453
- SACHIKO SUGIMOTO, KATSUYOSHI MATSUNAMI, HIDEAKI OTSUKA: "Medicinal Plants of Thailand. I Structures of Rheedeiosides A-D and cis-Entadamide A -D-Glucopyranoside from the Seed Kernels of Entada rheedei", CHEMICAL AND PHARMACEUTICAL BULLETIN, vol. 59, no. 4, 3 février 2011 (2011-02-03), - 3 février 2011 (2011-02-03), pages 446-471, XP002786918, Japan ISSN: 1347-5223, DOI: https://doi.org/10.1248/cpb.59.466 1248/cpb.59.466
- W.M KON? ET AL: "Traditional medicine in North C?te-d'Ivoire: screening of 50 medicinal plants for antibacterial activity", JOURNAL OF ETHNOPHARMACOLOGY, vol. 93, no. 1, 1 juillet 2004 (2004-07-01), pages 43-49, XP055070707, ISSN: 0378-8741, DOI: 10.1016/j.jep.2004.03.006
- INNGJERDINGEN K ET AL: "An ethnopharmacological survey of plants used for wound healing in Dogonland, Mali, West Africa", JOURNAL OF ETHNOPHARMACO, ELSEVIER IRELAND LTD, IE, vol. 92, no. 2-3, 1 janvier 2004 (2004-01-01), pages 233-244, XP009136859, ISSN: 0378-8741

## Description

La présente invention concerne un procédé d'obtention d'un extrait obtenu à partir d'une matière végétale et enrichi en métabolites d'intérêt, les extraits issus d'un tel procédé et les compositions comprenant de tels extraits, ainsi que leurs applications cosmétiques et dermocosmétiques.

Selon l'Organisation Mondiale de la Santé, les plantes qualifiées de "plante médicinale" pour leurs propriétés bénéfiques pour la santé humaine sont utilisées depuis des temps immémoriaux en médecine traditionnelle, notamment parmi les sociétés faiblement industrialisées pour lesquelles l'accès aux médicaments synthétiques modernes reste coûteux (Sofowora A. et al., Afr. J. Tradit. Complément Altern. Med., 2013, vol.10, pp.210-229).

L'une d'entre elles, l'espèce *Entada phaseoloides* qui appartient au genre "Entada" et plus largement à la famille botanique des Fabacées, trouve ainsi de longue date une utilisation dans les médecines non conventionnelles ayurvédique, chinoise, ou encore tribale (Deepa C. et al., Int. J. Curr. Res. Biosci. Plant Biol., 2017, vol.4, pp.92-99). Sa distribution est plutôt cosmopolite puisque la plante s'est primairement développée dans des zones tropicales chaudes telles les forêts côtières de pays bordant l'Océan Indien : Madagascar, Afrique du Sud, Inde, Australie, etc (Deepa C. et al., Int. J. Curr. Res. Biosci. Plant Biol., 2017, vol.4, pp. 92-99) . On la retrouve aussi en Asie tel dans la chaîne centrale et orientale de l'Himalaya et jusqu'à une altitude de 1300 mètres (Singh O. et al., J. Asian Natur. Products Res., 2011, vol.13, pp.682-687), ou encore en Chine sous précisément la dénomination *"Entada phaseoloides* (L.) Merrill" (Xiong H. et al., Acta Pharmaceutica Sinica, 2010, vol.45, pp.624-626 et références citées). La morphologie de la plante est celle d'une liane grimpante, porteuse après son inflorescence de grandes gousses pouvant atteindre jusqu'à 2 m de long et 13 cm de large. Chaque gousse contient entre 10 et 20 graines de couleur rouge brun, à l'enveloppe très dure, et dont la forme s'apparente à celle d'une lentille d'objectif pouvant aller jusqu'à 10 cm de diamètre (Sugimoto S. et al., J. Natur. Medecines, 2018, vol.72, pp.12-19).

Les vertus médicinales reconnues à la plante prise dans sa totalité avec ses principaux organes (tige, feuilles et graines) sont nombreuses et extrêmement variées : analgésique, anti-inflammatoire, anti-arthritique, antidiabétique, antioxydante, hépatoprotectrice, antimicrobienne, antispasmodique, antitussif, astringent, etc. Les expériences et études pharmacologiques menées à ce jour l'ont été toutefois principalement sur les graines du végétal (Deepa C. et al., Int. J. Curr. Res. Biosci. Plant Biol., 2017, vol.4, pp.92-99). Il est vrai d'une part qu'une fois leur chair blanchâtre simplement broyée et moulue, ces graines affichent un large champ d'utilisations : remède contre la jaunisse, la constipation, les morsures de serpent, les douleurs abdominales, mais aussi une exploitation comme stimulant de la pousse du cheveu, aphrodisiaque, vomitif, vermifuge, etc (Barua C.C. et al., Int. J. Res. Biosciences, 2015, vol.4, pp.88-97 ; Barua C.C. et al., J. Pharm. Pharmacol., 2014, vol.2, pp.1-18). D'autre part, l'analyse des constituants phytochimiques de la graine *d'Entada phaseoloides* révèle l'accumulation et le stockage de plusieurs métabolites secondaires d'intérêt (Shodhganga et al., 2005, adresse URL :
shodhganga.inflibnet.ac.in/bitstream/10603/42603/9/09-chapt er 4.pdf). Avant tout, il y est abondamment présent des saponines qui, chez les végétaux en général, s'avèrent être des glycosides huileux de triterpènes cycliques ou de stéroïdes, et qui, dans le cas *d'Entada phaseoloides* après une hydrolyse acide, libère une sapogénine cristalline, l'acide entagénique, et les glucosides arabinose et xylose. Il est tout particulièrement attribué, pour les saponines de graines *d'Entada phaseoloides,* une activité anti-inflammatoire significative, couplée à des activités antidiabétique et hypolipidémiante (Barua C.C. et al., J. Pharm. Pharmacol., 2014, vol.2, pp.1-18 et références citées). Ensuite, les graines *d'Entada phaseoloides* contiennent une multitude d'acides phénoliques et autres flavonoïdes, essentiellement sous forme de glycoconjugués telle l'entité "phaséoloidine" souvent citée dans la littérature sur la plante et qui, après caractérisation, s'est avérée être le dérivé 2-O- β-D-glucopyranoside de l'acide homogentisique (Barua A.K., Phytochem., 1988, vol.27, pp.3259-3261). Une telle richesse en composés phénoliques a d'ailleurs fait de ces graines, logiquement par la suite, une matière première de choix pour l'obtention d'antioxydants naturels puissants et nutritifs (Barua C.C. et al., Int. J. Pharm. Bio. Sci., 2015, vol.6, pp.366-376). Egalement et de manière originale à cette plante grimpante, il a été démontré la présence dans ces graines de structures thioamides appelées "entadamides", isolées et caractérisées pour la première fois par des universitaires japonais au milieu des années 1980 (Ikegami F. et al., Chem. Pharm. Bull., 1985, vol.33, pp.5153-5154 & Phytochem., 1987, vol.26, pp.1525-1526). Ces métabolites, au nombre précisément de deux structures identifiées dans les graines, sont appelés "entadamide A" et "entadamide B". Ils correspondent chimiquement aux composés "trans-N-(2-hydroxyéthyl)-3-méthylthio-propénamide" et "N-(2-hydroxyéthyl)-3,3-bis(méthylthio)-propénamide", et rapidement après leur caractérisation, tous deux ont été proposés comme nouveaux médicaments anti-inflammatoires après la mise en évidence d'un effet inhibiteur sur la lipoxygénase (Ikegami F. et al., Chem. Pharm. Bull., 1989, vol.37, pp.1932-1933).

En application topique sur la peau, un même bénéfice anti-inflammatoire est retrouvé à travers deux formulations, pommade et pâte, testées, contenant de la pulpe écrasée de graines d'*Entada phaseoloides.* Elles ont en effet démontré une efficacité au moins égale à celle d'un anti-inflammatoire notoire non stéroïdien, le diclofénac (Dawane J.S., N. Am. J. Med. Sci., 2011, vol.3, pp.513-517). Identiquement en matière de soin cutané mais concernant plus spécifiquement le seul entadamide A, il peut être signalé également la demande de brevet EP 3103436 déposée par la demanderesse où le composé "(E)-N-(2-hydroxyéthyl)-3-méthylthiopropénamide", soit une réplique synthétique de l'entadamide A, affiche dans les tests illustrant l'invention un large spectre d'activités protectrices contre le rayonnement solaire. L'entadamide A s'y trouve ainsi logiquement revendiqué dans une composition cosmétique ou dermocosmétique destinée à la photoprotection. A nouveau pour cet entadamide A mais aussi pour un "entadamide A-glucoside", un très récent article souligne leur action inhibitrice sur la production de mélanine, semblable à celle du composé de référence en la matière, l'arbutine (Sugimoto S. et al., J. Nat. Med., 2018, vol.72, pp.12-19). Ses auteurs prédisent même à l'ensemble des dérivés entadamides, tenant compte de leur structure jugée peu similaire avec les actuels "cosmetic drugs" disponibles commercialement, une prospective avantageuse avec la conclusion suivante : « *entadamides derivatives can be expected to be used as a new type of cosmetic* » (Sugimoto S. et al., J. Nat. Med., 2018, vol.72, pp.12-19).

Fort de ces constats, et en réponse à une demande croissante pour l'intégration d'actifs d'origine naturelle innovants, sûrs et efficaces dans les produits cosmétiques, l'intérêt de la demanderesse pour les dérivés entadamides s'est poursuivi après sa demande de brevet passée, mais nouvellement cette fois-ci à travers la conception d'un extrait végétal issu de graines *d'Entada* à la teneur optimale en de tels métabolites à visée cosmétique.

Pour cette plante, une connaissance limitante est que nombre des métabolites accumulés dans ses graines existent sous une forme essentiellement précurseur, parfois moins active biologiquement, avec leur conjugaison à un ou plusieurs glycosides de configuration ou de conformation spécifique (Kren V., Glycoscience, 2008, pp.2589-2644). Il s'agit ainsi des β-D-glucopyranose et β-D-glucopyranosyl-(1->3)-β-D-glucopyranose pour l'entadamide A (Xiong H. et al., Acta Pharmaceutica Sinica, 2010, vol.45, pp.624-626), du β-D-glucopyranose pour l'acide homogentisique (Barua A.K., Phytochem., 1988, vol.27, pp.3259-3261), ou encore de la N-acétylglucosamine retrouvée dans les saponines triterpènes (Iwamoto Y. et al., J. Nat. Med., 2012, vol.66, pp.321-328). Une autre difficulté est le constat d'une entadamide A sous forme glycosylée instable en solution dans l'eau et évoluant défavorablement vers la formation de sous-produits de structure mal définie dépourvus dès lors d'un quelconque intérêt cosmétique.

En conséquence, et compte tenu de ce qui précède, le problème technique que se propose de résoudre la présente invention est de développer à l'échelle industrielle un procédé d'obtention d'un extrait de graines d'*Entada* enrichi en entadamide A, précisément un entadamide A sous forme libre ou aglycone. Un tel procédé va donc impliquer une étape d'hydrolyse du glycoside précurseur, et la quête au final d'un extrait directement utilisable en cosmétique, c'est-à-dire sans aucune étape de séparation et donc de purification.

Le problème à résoudre dans la conception de l'extrait visé réside également dans la nécessité d'éviter l'hydrolyse concomitante d'autres constituants de l'extrait. Ainsi, la présence normalement attendue d'acide homogentisique résultant de l'hydrolyse simultanée de son précurseur glucosylé, à savoir la phaséoloidine doit être tout particulièrement évitée, car il est de connaissance acquise que cet acide-phénol, par ailleurs irritant cutané et oculaire selon les critères de classification du règlement européen n° 1272/2008, s'oxyde facilement en quinones qui polymérisent et forment ainsi des pigments peu solubles dans l'eau et de couleur très foncée (Consden R. et al., Biochem J., 1951, vol. 50, pp.274-278). Un extrait pourvu en acide homogentisique est donc instable dans le temps et aura tendance à évoluer spontanément vers la formation d'un précipité coloré rendant dès lors un tel extrait incompatible avec les contraintes imposées par la formulation cosmétique. Un autre écueil à éviter est enfin l'hydrolyse simultanée des saponines qui vont être présentes dans l'extrait, car elle va conduire à la perte des activités biologiques qui leur sont reconnues, ainsi qu'à la formation de composés triterpènes aglycones peu solubles dès lors dans l'eau et donc susceptibles une nouvelle fois de conduire à l'apparition d'un précipité dans l'extrait.

Pour atteindre ces objectifs, la demanderesse s'est originellement tournée vers des techniques d'hydrolyse chimique, pour constater alors qu'il n'était pas possible d'obtenir la sélectivité attendue présentée ci-avant. Elle s'est ensuite orientée vers des techniques d'hydrolyse enzymatique, connues de l'homme du métier pour être plus sélectives (Wang J. et al., African J. Biotech., 2011, vol.10, pp.14160-1466). L'exploitation d'hydrolases endogènes, notamment de glycosidases, dont la présence avait été rapportée dans certaines plantes de la famille des Fabacées à laquelle l'espèce *Entada phaseoloides* appartient (Sripuan T. et al., 1998, Proceedings of the twenty-four congress on Science and Technology of Thailand, Bangkok, Programme and abstracts, ISBN 974-86505-5-3, pp.638-639), est apparue comme une opportunité pour résoudre le problème technique posé par l'invention. D'une part, elle permet déjà d'éviter l'ajout d'enzymes hydrolytiques de source exogène pouvant être par la suite difficiles à éliminer de l'extrait final. D'autre part, une récente littérature a fait état spécifiquement de β-glucosidases endogènes dans plusieurs plantes cultivées pour leurs graines, et notamment de leur capacité à convertir de grandes quantités d'isoflavones glucosides en des isoflavones aglucones et *in fine* à augmenter la biodisponibilité de celles-ci pour leur exploitation en alimentaire (Fujita A. et al., Appl. Biochem. Biotechnol., 2015, vol.176, pp.1659-1672).

Sur cette base, la demanderesse a cherché à mettre à profit cette activité enzymatique endogène de végétaux à graines, pour parvenir à une hydrolyse originalement sélective. Les hydrolases de graines d'*Entada phaseoloides* se sont pourtant révélées, dans un premier temps, intrinsèquement peu sélectives. Dans un second temps cependant, et c'est le fondement de la présente invention, la demanderesse a découvert qu'une procédure séquentielle d'extraction permettait d'obtenir, selon un panel approprié de conditions opératoires, une hydrolyse enzymatique très fortement sélective des principaux métabolites glycoconjugés présents dans les graines d'*Entada phaseoloides,* avec une sélectivité objectivée par des différences de cinétique d'hydrolyse dépendantes de la nature du substrat.

En d'autres termes, les inventeurs de la présente invention ont mis en évidence que l'hydrolyse des formes β-D-glucopyranosides d'entadamide A et de la phaséoloidine par une β-glucosidase endogène pouvait, contrairement au préjugé technique, ne plus être concomitante, avec la mise en place d'une étape d'activation puis de désactivation enzymatique dans des conditions très précisément maîtrisées : solvant, concentration, température, pH et temps d'hydrolyse. En particulier selon ces conditions maîtrisées, les inventeurs de la présente invention ont découvert que l'introduction de certains solvants organiques dans le milieu d'hydrolyse à un stade précis du procédé permettait de stopper rapidement le processus d'hydrolyse enzymatique en inactivant, de manière irréversible, les glycosidases endogènes. En jouant ainsi sur la sensibilité respective des métabolites, il a été possible de limiter finalement l'hydrolyse aux seuls glycosides de l'entadamide A.

Concernant un état de la technique susceptible de se rapprocher de la solution au problème, l'art antérieur révèle tout d'abord une littérature assez abondante autour de l'Extraction Aqueuse Enzymatique dite "EAE" (Muniglia L. et al., Part of the "Green Chemistry and Sustainable Technology" book, 2014, pp.167-204). Celle-ci est essentiellement proposée à des fins de "chimie verte" et elle ne repose nullement, dans le cas d'un substrat végétal, sur l'hydrolyse sélective *in situ,* c'est-à-dire lors de l'extraction, de l'un des composés présents dans le végétal. La présente invention se démarque aussi de celle objet de la demande internationale WO95/10530 et de sa demande prioritaire concernant un procédé pour produire un extrait végétal enrichi en isoflavones aglucones à partir d'une ou plusieurs enzymes beta-glucosidase de telle sorte à convertir une majorité des isoflavones glucones en isoflavones aglucones. Contrairement à la présente invention, il n'est pas fait état d'une hydrolyse sélective de l'une en particulier des isoflavones glucones présentes dans le végétal.

La présente invention a donc pour premier objet un procédé de production d'un extrait de graines du genre Entada enrichi en entadamide A, comprenant les étapes suivantes :
(i) une phase d'activation des enzymes endogènes, préférentiellement d'une β-glucosidase, par dispersion, sous agitation mécanique, desdites graines dans un ratio massique graine/eau de 0,02 à 2 ;
(ii) une phase d'hydrolyse enzymatique de la dispersion obtenue à l'étape (i) par traitement thermique à une température comprise entre 25°C et 100°C et pendant une durée comprise entre 2 minutes et 12 heures, à un pH compris entre 4 et 8 ;
(iii) une phase d'inhibition de l'activité enzymatique dans la dispersion obtenue à l'étape (ii) par voie thermochimique, ladite phase comprenant successivement les étapes suivantes :
   a. l'addition d'un agent inhibant irréversiblement l'activité des enzymes endogènes dans un ratio volumique agent inhibiteur/eau compris entre 0,1 et 10 ;
   b. un traitement thermique du milieu réactionnel obtenu à l'étape (iii/a) à une température comprise entre 25°C et 75 °C et pendant une durée comprise entre 2 minutes et 12 heures ;
   c. une élimination de l'agent inhibiteur visé à l'étape (iii/a) sous pression réduite pour l'obtention d'un filtrat aqueux enrichi en entadamide A et contenant une phaséoloidine native non hydrolysée ;
(iv) l'addition d'un solvant cosmétiquement acceptable au filtrat aqueux obtenu à l'étape (iii/c), poursuivie par une étape d'ajustement du pH compris entre 3 et 7.

Par « extrait de graines du genre Entada enrichi en entadamide A » tel qu'obtenu au terme de l'étape (iv), il faut comprendre avant tout en premier lieu un extrait qui remplit au moins l'un des critères ci-après :
- un extrait enrichi en entadamide A sous forme libre (aglucone) et ne contenant plus d'entadamide A glucone ou ne contenant que des traces d'entadamide A glucone n'excédant pas une teneur massique de 0,2 mg/g (soit 200 ppm) ;
- un extrait enrichi en entadamide A, notamment par comparaison avec un extrait obtenu selon une extraction traditionnelle de type hydro-alcoolique qui ne permet la récupération que des très faibles quantités de métabolite aglucone ;
- un extrait comprenant une teneur massique comprise entre 1 et 5 mg/g en entadamide A, préférentiellement une teneur comprise entre envion 2 et 3 mg/g en entadamide A ;
- un extrait comprenant une teneur massique comprise entre 9 et envron 85 mg/g en phaséoloidine, préférentiellement une teneur comprise entre 30 et 60 mg/g en phaséoloidine ;
- un extrait dans lequel le rapport massique phaséoloidine/entadamide A est compris entre 15 et 25, préférentiellement le rapport massique est de 20 ;
- un extrait dans lequel le rapport massique entadamide A glucone/entadamide A est inférieur à 0,5, préférentiellement inférieur à 0,1 ;
- un extrait caractérisé par un taux de conversion de l'entadamide A glucone en entadamide A d'au moins 90%, préférentiellement de 99% ;
- un extrait n'ayant subi aucune étape de séparation par chromatographie.

En particulier, l'extrait de graines du genre Entada enrichi en entadamide A comprend une teneur massique comprise entre 1 et 5 mg/g en entadamide A, une teneur massique comprise entre 9 et 85 mg/g en phaséoloidine, et des traces d'entadamide A glucone n'excédant pas une teneur massique de 0,2 mg/g.

En second lieu, il faut comprendre par « extrait de graines du genre Entada enrichi en entadamide A » un extrait qui remplit au moins l'un des critères ci-après :
- un extrait ne contenant pas d'acide homogentisique ou ne contenant que des traces d'acide homogentisique n'excédant pas une teneur maximale de quelques dizaines de ppm, préférentiellement de 50 ppm (soit 0,05 mg/g) ;
- un extrait comprenant une teneur massique comprise entre 3 et 40 mg/g de saponines, préférentiellement une teneur comprise entre 10 et 20 mg/g de saponines ;
- un extrait renfermant une quantité optimale de phaséoloidine avec un rendement d'extraction en phaséoloidine d'au moins 90%, préférentiellement de 100%.

On entend par « graines du genre Entada » selon l'invention des graines entières ou décortiquées, germées ou non germées, concassées ou broyées, et éventuellement réduites à l'état de poudre. Suivant un mode de réalisation préféré de l'invention, ces graines sont issues de l'une ou plusieurs des espèces *Entada phaseoloides, Entada rheedei, Entada parvifolia, Entada pursaetha, Entada scandes, Entada gigas* et *Entada africana* au regard de la similitude annoncée de leurs phytoconstituents (Deepa C. et al., Int. J. Curr. Res. Biosci. Plant Biol., 2017, vol.4, pp.92-99 ; Sugimoto S. et al., J. Natur. Medecines, 2018, vol.72, pp.12-19 ; Abu Sufian Md. Et al., Int. Res. J. Pharm., 2015, vol.6, pp.411-414), de leurs synonymie ou encore de leur qualification de "sous-espèce" (Ohashi H. et al., Taiwania, 2010, vol.55, pp.43-53). Avantageusement, il s'agit de graines issues des espèces *Entada phaseoloides* ou *Entada rheedei.* Tout particulièrement, il s'agit de graines issues de l'espèce *Entada phaseoloides.*

Suivant un mode de réalisation préféré de l'invention, les graines sont décortiquées, concassées ou broyées, et éventuellement réduites à l'état de poudre.

Suivant un mode de réalisation préféré de l'invention, le ratio massique graine/eau de l'étape (i) est compris entre 0,1 et 1, préférentiellement le ratio massique est de 0,2.

Suivant un mode de réalisation préféré de l'invention, la température dans le traitement thermique de l'étape (ii) est comprise entre 45 et 60°C, préférentiellement la température est de 55°C.

Suivant un mode de réalisation préféré de l'invention, la durée du traitement thermique de l'étape (ii) est comprise entre 10 minutes et 1 heure, préférentiellement la durée est de 30 minutes.

Suivant un mode de réalisation préféré de l'invention, le pH est ajusté entre 4 et 6 pendant le traitement thermique de l'étape (ii).

Suivant un mode de réalisation préféré de l'invention, l'agent inhibiteur de l'activité des enzymes endogènes utilisé dans l'étape (iii/a) est un solvant organique miscible à l'eau, avantageusement une eau glucosée ou un alcool choisi parmi l'éthanol, le méthanol, le propanol et ses isomères, le butanol et ses isomères, le pentanol, l'hexanol, et leurs mélanges. Avantageusement, il s'agit de l'éthanol ou du méthanol. Tout particulièrement, il s'agit de l'éthanol.

Suivant un mode de réalisation préféré de l'invention, le ratio volumique agent inhibiteur/eau de l'étape (iii/a) est compris entre 0,5 et 5, préférentiellement le ratio volumique est de 1.

Suivant un mode de réalisation préféré de l'invention, la température dans le traitement thermique de l'étape (iii/b) est comprise entre 45 et 60°C, préférentiellement la température est de 55°C.

Suivant un mode de réalisation préféré de l'invention, la durée du traitement thermique de l'étape (iii/b) est comprise entre 10 minutes et 1 heure, préférentiellement la durée est de 30 minutes.

Suivant un mode de réalisation préféré de l'invention, le solvant cosmétiquement acceptable utilisé dans l'étape (iv) est un solvant glycolique, avantageusement choisi parmi le 1,3-propanediol, le 1,2-propanediol (propylène glycol), le méthylpropanediol, le phénoxypropanediol, le 1,2-butanediol (butylène glycol), le 1,3-butanediol, le 1,4-butanediol, le 2,3-butanediol, le 1,2-hexanediol, le 1,2-dihydroxyéthane (éthylène glycol), le diéthylène glycol, le dipropylène glycol, le triéthylène glycol, le tripropylène glycol, le diéthoxy diglycol, le pentylène glycol, l'hexylène glycol, le 1,2-octanediol (caprylyl glycol), la glycérine (glycérol), et leurs mélanges. Avantageusement, il s'agit du 1,3-propanediol, du 1,2-butanediol ou de la glycérine. Tout particulièrement, il s'agit du 1,3-propanediol.

Suivant un mode de réalisation préférée de l'invention, le pH d'ajustement de l'étape (iv) est de 4,5.

A titre d'exemple illustratif du premier objet de l'invention, un extrait de graines de l'espèce *Entada phaseoloides* enrichi en entadamide A est préparé selon un procédé comprenant les étapes suivantes :
(i) activation de la β-glucosidase endogène de 10g de graines décortiquées puis broyées d'*Entada phaseoloides* par leur mise en suspension sous agitation mécanique dans 50g d'eau ;
(ii) hydrolyse enzymatique de la suspension à une température de 55°C pendant 30 minutes ;
(iii) addition de 50 ml d'éthanol et milieu réactionnel porté à une température de 55°C pendant 30 autres minutes, poursuivie d'une filtration pour obtention d'un filtrat limpide ;
(iv) 100g de filtrat (« extrait brut ») obtenu à l'étape (iii) sont additionnés de 7,3g de 1,3-propanediol, qui, après concentration sous pression réduite et introduction de 50 ml d'eau, nouvelle concentration sous pression réduite, ajout de quelques gouttes d'HCl 6N et ajustement par 24,3g d'eau, fournissent un extrait hydroglycolique appelé « extrait de graines d'Entada » dans la formulation et tests 1 à 7 ci-après et renfermant, dans ces conditions :
   - 2,5 mg/g d'entadamide A ;
   - 43 mg/g de phaséoloidine ;
   - 10 à 15 mg/g de saponines ;
   - un rapport massique phaséoloidine/entadamide A inférieur à 20 ;
   - un rapport massique entadamide A glucone/entadamide A inférieur à 0,1 ;
   - une quantité non détectable d'acide homogentisique, du moins inférieure à 0,05 mg/g ;
   - une quantité non détectable d'entadamide A glucone, du moins inférieure à 0,05 mg/g.

La présente invention concerne en outre un extrait de graines du genre Entada enrichi en entadamide A susceptible d'être obtenu par le procédé tel que défini ci-dessus.

Un autre objet selon l'invention est un extrait de graines du genre Entada enrichi en entadamide A susceptible d'être obtenu par le procédé selon l'invention, pour son utilisation en cosmétique ou dermocosmétique puisque ledit extrait a présenté un faisceau de réponses avantageuses pour la peau, illustré par les tests 1 à 7 ci-après :
- une capacité à s'opposer à l'isomérisation photo-induite d'un agent photoprotecteur endogène, l'acide *trans*-urocanique, en conséquence une capacité à limiter les effets immunosuppresseurs de la composante ultra-violette B du rayonnement solaire (UV-B: λ 290-320 nm) [cf. test la ci-après]. A propos d'une telle activité photo-inhibitrice de l'extrait selon l'invention vis-à-vis de l'isomérisation photo-induite de l'acide *trans-*urocanique en acide cis-urocanique, il est à signaler, et c'est un autre aspect important de l'invention, la contribution notable, inconnue à ce jour à la connaissance de la demanderesse, de la phaséoloidine non hydrolysée, supérieure à minima à 25% dans une telle activité photoprotectrice contre le rayonnement UV-B [cf. test 1b ci-après] ;
- une puissante activité anti-oxydante pour ledit extrait, d'intérêt au plan cutané pour s'opposer à tout stress oxydatif générant des radicaux libres ou des espèces réactives dérivées de l'oxygène (O₂°⁻, H₂O₂, OH°, etc.). Un tel effet a été observé lorsque ledit extrait a été évalué dans le cadre des tests à l'ABTS (acide 2,2-azinobis-(3-éthylbenzothiazoline-6-sulfonique) [cf. test 2a ci-après] et au DPPH (1,1-diphényl-2-picrylhydrazyle) [cf. test 2b ci-après] ;
- une capacité à limiter la surproduction photo-induite d'une protéine, la galectine-7 ; un tel effet sur ce marqueur protéique constitue une autre indication de l'efficacité de l'extrait à s'opposer efficacement à l'immunosuppression induite par les UV-B [cf. test 3 ci-après] ;
- une capacité à limiter le relargage de médiateurs pro-inflammatoires cytokines de type IL-8 et TNF-alpha induits par le rayonnement UV-B, marqueurs désignés pour étudier les effets inflammatoires des UV-B [cf. tests 4 et 5 ci-après] ;
- une capacité à lutter efficacement contre d'autres composantes du rayonnement solaire, tels que le rayonnement ultraviolet-A (UV-A: λ 320-400 nm) ou le rayonnement visible (VIS: λ 400-700 nm), en particulier contre la lumière bleue (λ 380-470 nm) où ledit extrait, en raison de ses propriétés anti-oxydantes, est capable de s'opposer à la surproduction d'une métalloprotéinase matricielle, la MMP-1, induite par la lumière bleue et qui dégrade le collagène [cf. test 6 ci-après] ;
- une capacité à limiter, sur explants de peau humaine en culture, les dommages de l'ADN induits par la combinaison des rayonnements UV-B, UV-A et VIS et exprimés par la quantité de dimères de pyrimidine ("CPDs") [cf. test 7 ci-après] ;
- une capacité à protéger la bactérie commensale *Staphylococcus epidermidis* des dommages UVB-induits [cf. test 8 ci-après].

Pour l'un des tests susmentionnés (test 2), il est à signaler les valeurs comparatives entre l'extrait selon la présente invention et une réplique synthétique de l'entadamide A visée par la demande de brevet EP 3103436 déposée par la demanderesse.

Suivant un mode de réalisation préféré de l'invention, l'utilisation d'un extrait de graines du genre Entada enrichi en entadamide A susceptible d'être obtenu par le procédé selon l'invention concerne la prévention ou la lutte contre les effets du rayonnement solaire nocifs pour la peau, en particulier contre le rayonnement ultraviolet-B (UV-B: λ 290-320 nm), que ce soit pour renforcer l'efficacité de formules photo-protectrices appliquées topiquement ou plus généralement pour ralentir le photo-vieillissement.

Suivant un autre mode de réalisation préféré de l'invention et en raison des vertus anti-oxydantes de l'extrait selon l'invention, l'utilisation d'un tel extrait de graines du genre Entada enrichi en entadamide A susceptible d'être obtenu par le procédé selon l'invention concerne la prévention ou la lutte contre les signes cutanés résultant de stress tels que la pollution atmosphérique, le contact avec des xénobiotiques chimiques ou des atmosphères enfumées.

Un autre mode de réalisation préférée de l'invention concerne un extrait de graines du genre Entada enrichi en entadamide A susceptible d'être obtenu par le procédé selon l'invention, pour, et en raison de ses vertus anti-inflammatoires, son utilisation dermocosmétique dans le traitement de l'acné, de la séborrhée, de la rosacée ou de la dermatite atopique.

Un autre mode de réalisation préféré de l'invention est son utilisation cosmétique dans le maintien de l'homéostasie du microbiome à la surface de la peau, en raison des propriétés photo-protectrices et anti-oxydantes affichées par l'extrait de graines du genre Entada enrichi en entadamide A susceptible d'être obtenu par le procédé selon l'invention.

Enfin, un autre objet de l'invention concerne une composition, à usage cosmétique ou dermocosmétique, comprenant, à titre d'ingrédient actif principal, un extrait de graines du genre Entada enrichi en entadamide A tel que défini ci-dessus, en association avec un ou plusieurs adjuvants physiologiquement compatibles avec la peau ou les phanères.

La quantité d'extrait de graines du genre Entada enrichi en entadamide A tel que défini ci-dessus dans les compositions visées est comprise entre 1 % et 10 % en poids par rapport au poids total de la composition, de préférence entre 1,5 % et 5 % en poids.

Les compositions selon l'invention sont adaptées à une administration par voie topique cutanée, présentées sous toutes les formes normalement utilisées pour une telle administration. A titre indicatif et non limitatif, les compositions peuvent se présenter sous la forme de suspensions, lotions, crèmes, gels aqueux ou hydroalcooliques, poudres et émulsions multiples pouvant être éventuellement des microémulsions ou des nanoémulsions, etc.

Les compositions selon l'invention peuvent contenir comme adjuvant physiologiquement acceptable au moins un adjuvant connu de l'homme du métier et acceptable dans les domaines cosmétique ou dermocosmétique, choisi parmi les huiles, les cires, les élastomères de silicone, les tensioactifs, les co-tensioactifs, les épaississants et/ou gélifiants, les humectants, les émollients, les filtres solaires organiques ou inorganiques, les photostabilisants, les conservateurs, les colorants, les agents matifiants, les agents tenseurs, les séquestrants, les parfums, etc, et leurs mélanges.

Les compositions selon l'invention peuvent en outre comprendre un ou plusieurs actifs additionnels, choisis, sans que cette liste ne soit limitative, parmi les agents déglycants, les agents stimulant la synthèse de collagène ou d'élastine ou prévenant leur dégradation, les agents stimulant la synthèse de glycosaminoglycanes ou de protéoglycanes ou prévenant leur dégradation, les agents augmentant la prolifération cellulaire, les agents pro-pigmentants, les agents antioxydants ou anti-radicalaires ou anti-pollution, les agents hydratants, les agents drainants ou détoxifiants, les agents anti-inflammatoires, les agents desquamants, les agents apaisants et/ou anti-irritants, les agents agissant sur la microcirculation (anti-cernes), les agents renforçant les systèmes de défense cutanée, les agents favorables au renforcement de la fonction barrière, les agents stimulant le métabolisme cellulaire : croissance cellulaire, production de biomolécules utiles pour la peau telles que le collagène, les agents s'opposant aux effets néfastes du stress psychologique, les chaperons moléculaires, les agents limitant les inconvénients associés à une surproduction de sébum, les agents ciblant certaines pathologies cutanées telle que la dermatite atopique, la rosacée, les troubles de la cicatrisation, et leurs mélanges.

Tout particulièrement dans les utilisations et les compositions selon l'invention, l'extrait de graines du genre Entada enrichi en entadamide A susceptible d'être obtenu par le procédé selon l'invention est un extrait de graines décortiquées et broyées *d'Entada phaseoloides.*

La présente invention est illustrée ci-après, à titre purement indicatif, par l'exemple suivant de formulations de composition selon l'invention contenant un extrait de graines du genre Entada enrichi en entadamide A précité.
Formule A (crème) - application topique : photo-vieillissement (pourcentages massiques)

| | |
|---|---|
| Extrait de graines d'Entada phaseoloides | 2 % |
| Polyisobutène hydrogéné | 7 % |
| Myristate d'isobutyle | 3 % |
| Palmitate de cétyle | 7 % |
| Monostéarate d'éthylène glycol | 5 % |
| Laurate sorbitan | 2 % |
| Polysorbate 20 | 2 % |
| Carbomer (copolymère d'acrylate/acrylamide & huile minérale) | 0,3 % |
| Phénoxyéthanol | 0,5 % |
| Eau | qsp 100 % |

Formule B (gel) - application topique : anti-pollution (pourcentages massiques)

| | |
|---|---|
| Extrait de graines d'Entada phaseoloides | 4% |
| Carbomer (copolymère d'acrylate/acrylamide & huile minérale) | 1,5 % |
| Benzoate de sodium | 0,2 % |
| Acide sorbique | 1 % |
| 1,3-butanediol | 10 % |
| Glycérine | 5 % |
| Soude | 0,13 % |
| Phénoxyéthanol | 0,9 % |
| Eau | qsp 100 % |

La présente invention est également illustrée ci-après, à titre purement indicatif, par les tests suivants évoqués plus haut dans la description de l'invention (tests 1 à 7).

Les expérimentations des tests 3 à 5 ont toutes été menées sur des épidermes humains reconstruits à J17, appelés "EHR" (fournisseur : Skinethic^{®}), cultivés en milieu de maintenance puis irradiés à la dose de 300 mJ/cm² (lampe Waldmann^{®}) en présence ou non de l'extrait de graines d'Entada selon l'invention. Après 24h de culture, les surnageants ont été récoltés et les dosages protéiques ont alors été réalisés selon les protocoles ci-dessous.

### Test 1 : mise en évidence de la capacité d'un extrait de graines d'Entada phaseoloides à s'opposer à l'isomérisation photo-induite de l'acide trans-urocanique

Deux séries d'expérience ont été menées, la première à dose fixe en radiation UV-B de 300 mJ/cm² (test la), la seconde à dose variable en radiation UV-B (test 1b) et utilisant comme témoin une réplique synthétique de l'entadamide A, à savoir le composé "(*E*)-N-(2-hydroxyéthyl)-3-méthylpropénamide" objet de la demande de brevet EP 3103436.

Expérimentalement, le test a été réalisé dans un tampon citrate 0,01 M de pH 5,5, le pH légèrement acide étant plus en rapport avec l'acidité des couches cutanées superficielles dans lesquelles le marqueur acide *trans-*urocanique est localisé. Ainsi, les solutions à irradier (20mL), à savoir le contrôle avec ou sans l'extrait selon l'invention, ont été placées dans des boîtes de Petri en verre, non couvertes, puis soumises à température ambiante à une irradiation UV-B à l'aide d'un four à UV-B (LabOSI^{®}). Des aliquots (500 µL) ont alors été prélevés dans des vials HPLC pour être immédiatement stockés à l'abri de la lumière et à basse température (+4/+8°C), avant analyse par dosage HPLC. Des profils chromatographiques ont ainsi été obtenus, et il a été dosé la quantité résiduelle d'acide *trans*-urocanique.

Les résultats obtenus dans le cadre du test la sont rassemblés dans le tableau la suivant :

**Tableau la**

| **Composé** | **% d'acide *trans-*urocanique (à 300 mJ/cm²)** | **% d'augmentation d'acide *trans-*urocanique** |
|---|---|---|
| Acide trans-urocanique (contrôle non irradié) | 100 | N/A |
| Acide trans-urocanique (contrôle irradié) | 37 | N/A |
| Contrôle (irradié) + extrait de graines d'Entada à 2% | 53 | + 43% |
| Contrôle (irradié) + extrait de graines d'Entada à 3% | 56 | + 51% |
| Contrôle (irradié) + extrait de graines d'Entada à 5% | 64 | + 73% |

Les résultats du tableau la ci-dessus soulignent que l'extrait de graines d'Entada selon l'invention, dès la plus faible des concentrations testées, est efficace dans la prévention de la photo-isomérisation de l'acide *trans-*urocanique en acide *cis*-urocanique.

Expérimentalement concernant le test 1b, l'étude a été réalisée de manière quasi-identique au test la ci-avant, avec au préalable la vérification que dans les conditions expérimentales définies, l'acide *trans*-urocanique générait une quantité significative d'acide cis-urocanique que l'on peut mesurer par analyse HPLC.

La dose d'irradiation a été de 64 et de 128 mJ.cm⁻². Les résultats obtenus dans le cadre du test 1b sont rassemblés dans le tableau 1b suivant :

**Tableau 1b**

| **Composé** | **% d'inhibition de l'isomérisation trans -> cis de l'acide urocanique** | |
|---|---|---|
| | **64 mJ/cm²** | **128 mJ/cm²** |
| Témoin : entadamide A (1 mM) | 34 | 40 |
| Extrait de graines d'Entada (entadamide A (1 mM) + phaséoloidine) | 54 | 59 |

Les résultats du tableau 1b ci-dessus soulignent tout d'abord que l'extrait de graines d'Entada selon l'invention est efficace dans la protection de l'acide *trans*-urocanique vis-à-vis de l'irradiation UV-B, avec une capacité à limiter sa photo-isomérisation. Ensuite, ces mêmes résultats mettent en évidence la contribution importante de la phaséoloidine dans ce pouvoir photoprotecteur de l'extrait.

### Test 2 : mise en évidence de l'activité antioxydante d'un extrait de graines d'Entada phaseoloides

Deux séries d'expérience ont été menées, la première avec le test à l'ABTS ou acide 2,2-azinobis-(3-ethylbenzothiazoline-6-sulfonique (test 2a), la seconde avec le test au DPPH ou 1,1-diphényl-2-picrylhydrazyle (test 2b). L'acide ascorbique a été utilisé en tant qu'antioxydant de référence.

Expérimentalement selon le test à l'ABTS, une solution mère de sel d'ABTS (sel de diammonium d'acide 2,2'-azino-bis(3-éthylbenzothiazoline-6-sulfonique) oxydé a été obtenue préalablement en mélangeant volume à volume 7 mM de sel de diammonium d'acide 2,2'-azinobis(3-éthylbenzothyazoline-6-sulfonique avec 2,45 mM de persulfate de potassium pendant 16 heures. Cette solution a ensuite été diluée dans de l'eau pour obtenir, dans les conditions du test, une absorbance de 0,5 à 734 nm. Dans chaque puits d'une micro plaque 96 puits, ont été placés 100L d'une solution de l'extrait selon l'invention à évaluer et 150 µl de solution d'ABTS oxydé. Après 6 minutes, l'absorbance a été mesurée à 734 nm.

Les résultats obtenus sont rassemblés dans le tableau 2a suivant :

**Tableau 2a**

| **Composé** | **CE₅₀** |
|---|---|
| extrait d'Entada | 74 mg/l (soit 9,6 µM équivalent phaséoloidine) |
| phaséoloidine | 8,3 µM |
| acide ascorbique | 24 µM |

Expérimentalement selon le test au DPPH, dans chaque puits d'une micro plaque 96 puits, ont été placés 100µL d'une solution de l'actif à évaluer et 100 µl d'une solution éthanolique de 1,1-diphényl-2-picrylhydrazyle (DPPH-Aldrich D913-2) à 200µM. Après 30 minutes, l'absorbance a été mesurée à 517 nm.

Les résultats obtenus sont rassemblés dans le tableau 2b suivant :

**Tableau 2b**

| **Composé** | **EC 50** |
|---|---|
| extrait d'Entada | 0,84 mg/l (soit 109 µM équivalent phaséoloidine) |
| phaséoloidine | 540 µM |
| acide ascorbique | 25 µM |

Les résultats des tableaux 2a et 2b soulignent que la phaséoloidine est un antioxydant puissant proche de l'acide ascorbique, elle confère à l'extrait une activité exploitable dans le cadre d'une application cosmétique.

### Test 3 : mise en évidence de la capacité d'un extrait de graines d'Entada phaseoloides à limiter la sécrétion d'une protéine, la qalectine-7

Expérimentalement, après irradiation et récolte des surnageants, le dosage de la galectine-7, protéine apoptotique, a été réalisé à l'aide d'un kit ELISA (human Galectine-7 R&D System DY13339, fournisseur : Biotechne). Les anticorps de capture anti-galectine-7 ont tout d'abord été fixés au fond de puits d'une plaque de 96 puits. Puis, après lavage et blocage, 100µl des différents surnageants de culture des EHR récoltés ont été déposés dans les puits. Les molécules de galectine-7 présentes dans les échantillons se sont ainsi fixées spécifiquement aux anticorps de capture et celles qui ne se sont pas fixées ont été éliminées lors d'un rinçage. L'anticorps secondaire de détection (porteur de sites biotinylés) a ensuite été ajouté dans les puits. Après lavage, la streptavidine-HRP, protéine à l'affinité haute pour la biotine, a été ajoutée et est venue se fixer sur les sites biotinylés de l'anticorps secondaire. Après un dernier rinçage pour éliminer l'excédent de Streptavidine-HRP, le substrat de la peroxydase a été ajouté et la réaction a été bloquée avec une solution contenant de l'acide sulfurique au bout de vingt minutes. L'intensité colorimétrique a été mesurée par spectrophotométrie à 450 nm.

Les résultats obtenus sont rassemblés dans le tableau 3 suivant :

**Tableau 3**

| **Composé** | **% de galectine-7 sécrétée** | **% d'inhibition de la sécrétion de qalectine-7** |
|---|---|---|
| Contrôle (EHR non irradiés) | 100 | N/A |
| Contrôle (EHR irradiés) | 1084 | N/A |
| Contrôle (EHR irradiés) + extrait de graines d'Entada à 2,5% | 301 | - 72% |

Les résultats du tableau 3 soulignent que sous une dose de rayonnement UV-B, l'extrait de graines d'Entada selon l'invention est capable de réduire de plus de 70% la quantité de galectine-7 sécrétée par les épidermes humains reconstruits.

### Test 4 : mise en évidence de la capacité d'un extrait de graines d'Entada phaseoloides à limiter le relargage de médiateurs pro-inflammatoires de type IL-8

Expérimentalement, après irradiation et récolte des surnageants, le dosage de la cytokine IL-8, synonyme d'une augmentation de l'inflammation sous UV-B, a été réalisé grâce à un kit ELISA de type « Human CXCL8/IL-8 Immunoassay D8000C » (fournisseur : Biotechne). Une technique identique au dosage de galectine-7 a été utilisée.

Les résultats obtenus sont rassemblés dans le tableau 4 suivant :

**Tableau 4**

| **Composé** | **% de IL-8 sécrétée** | **% d'inhibition de la sécrétion de IL-8** |
|---|---|---|
| Contrôle (EHR non irradiés) | 100 | N/A |
| Contrôle (EHR irradiés) | 1213 | N/A |
| Contrôle (EHR irradiés) + extrait de graines d'Entada à 1,5% | 221 | - 80% |

Les résultats du tableau 4 soulignent que sous une dose de rayonnement UV-B, l'extrait de graines d'Entada selon l'invention est capable de diminuer fortement la sécrétion d'IL-8.

### Test 5 : mise en évidence de la capacité d'un extrait de graines d'Entada phaseoloides à limiter le relargage de médiateurs inflammatoires (TNF alpha)

Expérimentalement, après irradiation et récolte des surnageants, le dosage de la cytokine TNF-alpha, cytokine pro-inflammatoire jouant un rôle important dans les réactions immunitaires et ayant une place prépondérante dans l'immunosuppression photo-induite, a été réalisé grâce à un kit ELISA de type « Quantikine Human TNF-alpha Immunoassay DTA00C » (fournisseur : Biotechne), et avec la même technique d'analyse que pour la galectine-7 et l'IL-8.

Les résultats obtenus sont rassemblés dans le tableau 5 suivant :

**Tableau 5**

| **Composé** | **% de TNF alpha sécrétée** | **% d'inhibition de la sécrétion de TNF alpha** |
|---|---|---|
| Contrôle (EHR non irradiés) | 100 | N/A |
| Contrôle (EHR irradiés) | 229 | N/A |
| Contrôle (EHR irradiés) + extrait de graines d'Entada à 1,5% | 165 | - 28% |

Les résultats du tableau 5 soulignent que sous une dose de rayonnement UV-B, l'extrait de graines d'Entada selon l'invention est capable de diminuer la sécrétion de TNF alpha.

### Test 6 : mise en évidence de la capacité d'un extrait de graines d'Entada phaseoloides à limiter la surexpression de la métalloprotéinase MMP-1 induite par le rayonnement bleu

Des fibroblastes humains primaires ("NHDF") ont été isolés à partir d'une plastie mammaire provenant d'une patiente de 17 ans. Les NHDF ont été cultivés dans du milieu DMEM additionné de 4,5 g/l de glucose et 10 % de SVF. Ils ont été maintenus dans une atmosphère à 37°C et 10 % de CO₂. A J-3, les fibroblastes ont été ensemencés en plaques 24 puits à raison de 15000 cellules/cm². A J-1, les NHDF ont été incubés à 37°C et 10 % de CO₂ pendant 24 h avec l'extrait de graines d'Entada selon l'invention. A J0, les traitements ont été renouvelés et les cellules ont été exposées à une dose de 30 J/cm² de lumière bleue (lampe Waldmann^{®}, avec ampoule fluocompacte LIGHTTECH LTC 36W/2G11 CL 380-470 nm). Après irradiation, les cellules ont été réincubées à 37°C et 10 % de CO₂ pendant 24 h supplémentaires et les surnageants de culture ont été récoltés afin de réaliser un dosage ELISA de la métalloprotéinase MMP-1 (kit Abcam, ab100603). Les résultats obtenus ont été normalisés par rapport à la quantité de cellules par condition et soumis à une analyse statistique en triplicate. La N-acétyl-cystéine ("NAC") a été utilisée en tant qu'anti-oxydant de référence.

Les résultats obtenus sont rassemblés dans le tableau 6 suivant :

**Tableau 6**

| **Composé** | **% de MMP-1 secrétée** | **% d'inhibition de la sécrétion de MMP-1** |
|---|---|---|
| Contrôle (NHDF non irradiés) | 100 | N/A |
| Contrôle (NHDF irradiés) | 214 | N/A |
| Contrôle (NHDF irradiés) + N-acétyl-L-cystéine (NAC) 5mM | 61 | - 71% |
| Contrôle (NHDF irradiés) + extrait de graines d'Entada à 0,05% | 46 | - 79% |

Les résultats du tableau 6 soulignent une efficacité de l'extrait de graines d'Entada selon l'invention comparable à celle de la N-acétyl-cystéine.

### Test 7 : mise en évidence de la capacité d'un extrait de graines d'Entada phaseoloides à limiter, sur explants de peau humaine en culture, les dommages de l'ADN induits par la combinaison des rayonnement UV-B, UV-A et VIS

La mise en évidence a été évaluée à l'aide de la technique d'immunomarquage *in situ* sur des explants de peau humaine en culture (peau humaine issue d'un scalp de la zone temporale de femmes de type caucasien âgées de 42 et 56 ans), après réalisation au scalpel de carrés de peau d'environ 1 cm². La mise en culture des explants a consisté à les placer dans un milieu de croissance "DMEM" contenant 4,5g/L de glucose, supplémenté avec des antibiotiques (pénicilline-streptomycine-amphotéricine) et traité topiquement avec l'extrait de graines d'Entada selon l'invention à la concentration de 2,5%, et ce pendant 48 heures à 37°C et 5% CO₂.

Après application de l'extrait, les explants ont été irradiés dans un simulateur solaire (SUNTEST CPS+). Après irradiation, le traitement a été renouvelé immédiatement et les explants ont été mis en culture pendant 24 heures. A la fin du temps de culture, les explants ont été congelés puis des coupes transversales de chaque explant de 5 µm d'épaisseur ont été obtenues à l'aide d'un cryotome. Un immunomarquage des dimères de pyrimidine ("CPDs") a été réalisé à l'aide d'anticorps spécifiques. Chaque coupe ou section de peau a alors été observée à l'aide d'un microscope à épifluorescence, avec prise de trois photographies au grossissement x20 pour chaque section, puis une mesure de la quantité globale de fluorescence du marquage a été réalisée à l'aide d'un logiciel d'analyse d'image. Les résultats de chaque condition ont été comparés à la condition témoin.

Les résultats obtenus sont rassemblés dans le tableau 7 suivant :

**Tableau 7**

| **Composé** | **Qté globale de fluorescence(UA)** | **% de diminution des CPDs** |
|---|---|---|
| Contrôle (explants non irradiés) | 921116 | N/A |
| Contrôle (explants irradiés) | 4987094 | N/A |
| Contrôle (explants irradiés) + extrait de graines d'Entada à 2,5% | 1915520 | - 61% |

Les résultats du tableau 7 soulignent que sous une dose de rayonnement solaire, l'extrait de graines d'Entada selon l'invention est capable de limiter notablement la formation de dimères de pyrimidine (CPDs) induits par le rayonnement solaire.

### Test 8 : test de protection du microbiote cutané avec une mise en évidence de la capacité d'un extrait de graines d'Entada phaseoloides à protéger la bactérie commensale Staphylococcus epidermidis des dommages UVB-induits

Expérimentalement, les tests ont été réalisés *in tubo.* La souche bactérienne *Staphylococcus epidermidis* (Alliance bioexpertise ATCC 12228) a été soumise à une irradiation UV-B de 100, 150 et 300 mJ/cm2 (lampe Waldmann, irradiance 0,73mW/cm²), en présence ou non de l'extrait d'intérêt à 1%, 1,5% et 2,5%.

100µl de suspension ont ensuite été déposés sur gélose TSA (Biomérieux, ref 43011) puis incubés à 37°C en conditions de culture aérobies. Un dénombrement par comptage manuel de colonies bactériennes a été effectué 24h après. Les résultats sont exprimés en pourcentage de CFU (Unités Formant Colonies) par rapport au contrôle non irradié, représentatifs de la viabilité bactérienne.

Les résultats sont présentés dans le tableau 8 suivant :

**Tableau 8**

| | **% de CFU (viabilité bactérienne)** | | |
|---|---|---|---|
| **Composé** | **Non irradié** | **100mJ/cm²** | **150mJ/cm²** |
| ***S.epidermidis* non traité** | 100 | 77 | 49 |
| ***S.epidermidis* + extrait d'Entada 1%** | 99 | 89 (*+16%*) | 73 (*+49%*) |
| ***S.epidermidis* + extrait d'Entada 1,5%** | 98 | 95 (*+23%*) | 89 (*+82%*) |
| ***S. epidermidis** +* **extrait d'Entada 2,5%** | 97 | 97 (*+26%*) | 94 (+*92%*) |

Les résultats du tableau 8 soulignent que sous rayonnement UV-B, l'extrait de graines d'Entada selon l'invention est capable de protéger de la mort cellulaire la bactérie principale composante du microbiome cutané, *staphylococcus epidermidis.*

## Revendications

1. Procédé de production d'un extrait de graines du genre Entada enrichi en entadamide A, comprenant les étapes suivantes :
(i) une phase d'activation des enzymes endogènes, préférentiellement d'une β-glucosidase, par dispersion, sous agitation mécanique, desdites graines, dans un ratio massique graine/eau de 0,02 à 2 ;
(ii) une phase d'hydrolyse enzymatique de la dispersion obtenue à l'étape (i) par traitement thermique à une température comprise entre 25°C et 100°C et pendant une durée comprise entre 2 minutes et 12 heures, à un pH compris entre 4 et 8 ;
(iii) une phase d'inhibition de l'activité enzymatique dans la dispersion obtenue à l'étape (ii) par voie thermochimique, ladite phase comprenant successivement les étapes suivantes :
a. l'addition d'un agent inhibant irréversiblement l'activité des enzymes endogènes dans un ratio volumique agent inhibiteur/eau compris entre 0,1 et 10 ;
b. un traitement thermique du milieu réactionnel obtenu à l'étape (iii/a) à une température comprise entre 25°C et 75 °C et pendant une durée comprise entre 2 minutes et 12 heures ;
c. une élimination de l'agent inhibiteur visé à l'étape (iii/a) sous pression réduite pour l'obtention d'un filtrat aqueux, enrichi en entadamide A, et contenant une phaséoloidine native non hydrolysée ;
(iv) l'addition d'un solvant cosmétiquement acceptable au filtrat aqueux obtenu à l'étape (iii/c), poursuivie par une étape d'ajustement du pH compris entre 3 et 7.

2. Procédé de production d'un extrait de graines du genre Entada enrichi en entadamide A selon la revendication 1, dans lequel les graines sont issues de l'une ou plusieurs des espèces *Entada phaseoloides, Entada rheedei, Entada parvifolia, Entada pursaetha, Entada scandes, Entada gigas* et *Entada africana.*

3. Procédé de production d'un extrait de graines du genre Entada enrichi en entadamide A selon l'une quelconque des revendications 1 à 2, dans lequel les graines sont issues de l'espèce *Entada phaseoloides.*

4. Procédé de production d'un extrait de graines du genre Entada enrichi en entadamide A selon l'une quelconque des revendications 1 à 3, dans lequel ledit extrait comprend, au terme de ladite étape (iv), une teneur massique comprise entre 1 et 5 mg/g en entadamide A, une teneur massique comprise entre 9 et 85 mg/g en phaséoloidine, et des traces d'entadamide A glucone n'excédant pas une teneur massique de 0,2 mg/g.

5. Procédé de production d'un extrait de graines du genre Entada enrichi en entadamide A selon l'une quelconque des revendications 1 à 4, dans lequel le rapport massique phaséoloidine/entadamide A est compris entre 15 et 25, et le rapport massique entadamide A glucone/entadamide A est inférieur à 0,5.

6. Procédé de production d'un extrait de graines du genre Entada enrichi en entadamide A selon l'une quelconque des revendications 1 à 5, dans lequel l'agent inhibiteur de l'activité des enzymes endogènes de l'étape (iii/a) est un solvant organique miscible à l'eau, avantageusement une eau glucosée ou un alcool choisi parmi l'éthanol, le méthanol, le propanol et ses isomères, le butanol et ses isomères, le pentanol, l'hexanol, et leurs mélanges.

7. Procédé de production d'un extrait de graines du genre Entada enrichi en entadamide A selon la revendication 6, dans lequel ledit agent est l'éthanol.

8. Procédé de production d'un extrait de graines du genre Entada enrichi en entadamide A selon l'une quelconque des revendications 1 à 7, dans lequel le solvant cosmétiquement acceptable utilisé dans l'étape (iv) est un solvant glycolique, avantageusement choisi parmi le 1,3-propanediol, le 1,2-propanediol, le méthylpropanediol, le phénoxypropanediol, le 1,2-butanediol, le 1,3-butanediol, le 1,4-butanediol, le 2,3-butanediol, le 1,2-hexanediol, le 1,2-dihydroxyéthane, le diéthylène glycol, le dipropylène glycol, le triéthylène glycol, le tripropylène glycol, le diéthoxy diglycol, le pentylène glycol, l'hexylène glycol, le 1,2-octanediol, la glycérine, et leurs mélanges.

9. Procédé de production d'un extrait de graines du genre Entada enrichi en entadamide A selon la revendication 8, dans lequel ledit solvant est le 1,3-propanediol.

10. Extrait de graines du genre Entada enrichi en entadamide A susceptible d'être obtenu par la mise en oeuvre du procédé selon l'une quelconque des revendications 1 à 9.

11. Extrait de graines du genre Entada enrichi en entadamide A selon la revendication 10, qui comprend une teneur massique comprise entre 1 et 5 mg/g en entadamide A, une teneur massique comprise entre 9 et 85 mg/g en phaséoloidine, et des traces d'entadamide A glucone n'excédant pas une teneur massique de 0,2 mg/g.

12. Extrait de graines du genre Entada enrichi en entadamide A selon l'une quelconque des revendications 10 et 11, pour utilisation pour la prévention ou la lutte contre les effets du rayonnement solaire nocifs pour la peau, en particulier contre le rayonnement ultraviolet-B.

13. Extrait de graines du genre Entada enrichi en entadamide A selon l'une quelconque des revendications 10 et 11, pour utilisation pour la prévention ou la lutte contre les signes cutanés résultant de la pollution atmosphérique, du contact avec des xénobiotiques chimiques ou des atmosphères enfumées.

14. Utilisation cosmétique non-thérapeutique d'un extrait de graines du genre Entada enrichi en entadamide A selon l'une quelconque des revendications 10 et 11, comme agent utile au maintien de l'homéostasie du microbiome présent à la surface de la peau.

15. Extrait de graines du genre Entada enrichi en entadamide A selon l'une quelconque des revendications 10 et 11, pour utilisation dermocosmétique dans le traitement de l'acné, de la séborrhée, de la rosacée ou de la dermatite atopique.

16. Composition, à usage cosmétique ou dermocosmétique, qui comprend, à titre d'ingrédient actif principal, un extrait de graines du genre Entada enrichi en entadamide A selon l'une quelconque des revendications 10 et 11, en association avec un ou plusieurs adjuvants physiologiquement compatible avec la peau ou les phanères, et dans laquelle la quantité dudit extrait est comprise entre 1 % et 10 % en poids par rapport au poids total de la composition.

## Patentansprüche

1. Verfahren zur Herstellung eines Extraktes aus Samen der Gattung Entada, angereichert mit Entadamid A, das die folgenden Schritte umfasst:
(i) eine Phase der Aktivierung der endogenen Enzyme, vorzugsweise einer β-Glucosidase, durch Dispergieren der Samen unter mechanischem Rühren, in einem Samen/Wasser-Massenverhältnis von 0,02 bis 2,
(ii) eine Phase einer enzymatischen Hydrolyse der in Schritt (i) erhaltenen Dispersion durch Wärmebehandlung bei einer Temperatur im Bereich zwischen 25 °C und 100 °C sowie für eine Dauer zwischen 2 Minuten und 12 Stunden, bei einem pH-Wert im Bereich zwischen 4 und 8,
(iii) eine Phase des Hemmens der enzymatischen Aktivität in der in Schritt (ii) erhaltenen Dispersion auf thermochemischem Weg, wobei die Phase nacheinander die folgenden Schritte umfasst:
a. das Zugeben eines Mittels, das die Aktivität der endogenen Enzyme irreversibel hemmt, in einem Volumenverhältnis von Inhibitor/Wasser zwischen 0,1 und 10,
b. eine Wärmebehandlung des in Schritt (iii/a) erhaltenen Reaktionsmediums, bei einer Temperatur im Bereich zwischen 25 °C und 75 °C sowie für eine Dauer zwischen 2 Minuten und 12 Stunden,
c. ein Entfernen des in Schritt (iii/a) genannten Inhibitors unter vermindertem Druck, um ein wässriges Filtrat zu erhalten, das mit Entadamid A angereichert ist und ein nicht-hydrolysiertes natives Phaseolidin enthält,
(iv) das Zugeben eines kosmetisch akzeptablen Lösungsmittels zu dem in Schritt (iii/c) erhaltenen wässrigen Filtrat, woran sich ein Schritt des Einstellens des pH-Wertes zwischen 3 und 7 anschließt.

2. Verfahren zur Herstellung eines Extraktes aus Samen der Gattung Entada, angereichert mit Entadamid A, nach Anspruch 1, bei dem die Samen von einer oder mehreren der Arten *Entada phaseoloides, Entada rheedei, Entada parvifolia, Entada pursaetha, Entada scandes, Entada gigas* und *Entada africana* stammen.

3. Verfahren zur Herstellung eines Extraktes aus Samen der Gattung Entada, angereichert mit Entadamid A, nach einem der Ansprüche 1 bis 2, bei dem die Samen von der Art *Entada phaseoloides* stammen.

4. Verfahren zur Herstellung eines Extraktes aus Samen der Gattung Entada, angereichert mit Entadamid A, nach einem der Ansprüche 1 bis 3, bei dem der Extrakt am Ende des Schrittes (iv) einen Massengehalt im Bereich zwischen 1 und 5 mg/g Entadamid A, einen Massengehalt im Bereich zwischen 9 und 85 mg/g Phaseoloidin sowie Spuren von Entadamid-A-Glucon, die einen Massengehalt von 0,2 mg/g nicht überschreiten, umfasst.

5. Verfahren zur Herstellung eines Extraktes aus Samen der Gattung Entada, angereichert mit Entadamid A, nach einem der Ansprüche 1 bis 4, bei dem das Massenverhältnis Phaseolidin/Entadamid A zwischen 15 und 25 liegt und das Massenverhältnis Entadamid A-Glucon/Entadamid A kleiner als 0,5 ist.

6. Verfahren zur Herstellung eines Extraktes aus Samen der Gattung Entada, angereichert mit Entadamid A, nach einem der Ansprüche 1 bis 5, bei dem das Mittel, das die Aktivität der endogenen Enzyme aus Schritt (iii/a) hemmt, ein organisches Lösungsmittel ist, das mit Wasser mischbar ist, vorteilhafterweise ein Glucosewasser oder ein Alkohol, ausgewählt aus Ethanol, Methanol, Propanol und seinen Isomeren, Butanol und seinen Isomeren, Pentanol, Hexanol und deren Mischungen.

7. Verfahren zur Herstellung eines Extraktes aus Samen der Gattung Entada, angereichert mit Entadamid A, nach Anspruch 6, wobei das Mittel Ethanol ist.

8. Verfahren zur Herstellung eines Extraktes aus Samen der Gattung Entada, angereichert mit Entadamid A, nach einem der Ansprüche 1 bis 7, bei dem das kosmetisch akzeptable Lösungsmittel, welches in Schritt (iv) verwendet wird, ein glykolisches Lösungsmittel ist, vorteilhafterweise ausgewählt aus 1,3-Propandiol, 1,2-Propandiol, Methylpropandiol, Phenoxypropandiol, 1,2-Butandiol, 1,3-Butandiol, 1,4-Butandiol, 2,3-Butandiol, 1,2-Hexandiol, 1,2-Dihydroxyethan, Diethylenglycol, Dipropylenglycol, Triethylenglycol, Tripropylenglycol, Diethoxydiglycol, Pentylenglycol, Hexylenglycol, 1,2-Octandiol, Glycerin und deren Mischungen.

9. Verfahren zur Herstellung eines Extraktes aus Samen der Gattung Entada, angereichert mit Entadamid A, nach Anspruch 8, wobei das Lösungsmittel 1,3-Propandiol ist.

10. Extrakt aus Samen der Gattung Entada, angereichert mit Entadamid A, der geeignet ist, durch das Durchführen des Verfahrens nach einem der Ansprüche 1 bis 9 erhalten zu werden.

11. Extrakt aus Samen der Gattung Entada, angereichert mit Entadamid A, nach Anspruch 10, der einen Massengehalt im Bereich zwischen 1 und 5 mg/g Entadamid A, einen Massengehalt im Bereich zwischen 9 und 85 mg/g Phaseoloidin sowie Spuren von Entadamid-A-Glucon, die einen Massengehalt von 0,2 mg/g nicht überschreiten, umfasst.

12. Extrakt aus Samen der Gattung Entada, angereichert mit Entadamid A, nach einem der Ansprüche 10 und 11, zur Verwendung für die Vorbeugung der oder den Kampf gegen die die Haut schädigenden Wirkungen der Sonnenstrahlung, insbesondere gegen die UV-B-Strahlung.

13. Extrakt aus Samen der Gattung Entada, angereichert mit Entadamid A, nach einem der Ansprüche 10 und 11, zur Verwendung für die Vorbeugung der oder den Kampf gegen die Hauterscheinungen, die aus der Luftverschmutzung, dem Kontakt mit chemischen Xenobiotika oder rauchiger Luft resultieren.

14. Nicht-therapeutische kosmetische Verwendung eines Extraktes aus Samen der Gattung Entada, angereichert mit Entadamid A, nach einem der Ansprüche 10 und 11, als Mittel, das zur Aufrechterhaltung der Homöostase des an der Hautoberfläche vorhandenen Mikrobioms nützlich ist.

15. Extrakt aus Samen der Gattung Entada, angereichert mit Entadamid A, nach einem der Ansprüche 10 und 11, zur dermokosmetischen Verwendung bei der Behandlung von Akne, Seborrhoe, Rosacea oder atopischer Dermatitis.

16. Zusammensetzung zur kosmetischen oder dermokosmetischen Verwendung, die als Hauptwirkbestandteil einen Extrakt aus Samen der Gattung Entada, angereichert mit Entadamid A, nach einem der Ansprüche 10 und 11 in Kombination mit einem oder mehreren mit der Haut oder den Hautanhangsgebilden physiologisch verträglichen Hilfsstoffen umfasst, und bei der die Menge des Extraktes zwischen 1 und 10 Gew.-%, bezogen auf das Gesamtgewicht der Zusammensetzung liegt.

## Claims

1. A process for producing an extract of seeds of the genus Entada enriched in entadamide A, comprising the following steps:
(i) an activation phase of endogenous enzymes, preferably of a β-glucosidase, by dispersion of said seeds under mechanical stirring in a seed/water weight ratio of 0.02 to 2;
(ii) a phase of enzymatic hydrolysis of the dispersion obtained in step (i) by heat treatment at a temperature between 25 and 100°C and for a time period of between 2 minutes and 12 hours, at a pH of between 4 and 8;
(iii) an inhibition phase of the enzymatic activity in the dispersion thermo-chemically obtained in step (ii), said phase successively comprising the following steps:
a) the addition of an agent irreversibly inhibiting the activity of the endogenous enzymes in an inhibitory agent/water volume ratio of between 0.1 and 10;
b) a heat treatment of the reaction medium obtained in step (iii/a) at a temperature of between 25 and 75°C and for a time period of between 2 minutes and 12 hours;
c) removal of the inhibitory agent referred to in step (iii/a) under reduced pressure in order to obtain an aqueous filtrate, enriched in entadamide A, and containing a native non-hydrolyzed phaseoloidin;
(iv) the addition of a cosmetically acceptable solvent to the aqueous filtrate obtained in step (iii/c), followed by a step of adjustment of the pH between 3 and 7.

2. The process for producing an extract of seeds of the genus Entada enriched in entadamide A according to claim 1, wherein the seeds are derived from one or more of the species *Entada phaseoloides, Entada rheedei, Entada parvifolia, Entada pursaetha, Entada scandes, Entada gigas* and *Entada africana.*

3. The process for producing an extract of seeds of the genus Entada enriched in entadamide A according to any one of claims 1 to 2, wherein the seeds are derived from the species *Entada phaseoloides.*

4. The process for producing an extract of seeds of the genus Entada enriched in entadamide A according to any one of claims 1 to 3, wherein said extract comprises after said step (iv) a weight content of between 1 and 5 mg/g of entadamide A, a weight content of between 9 and 85 mg/g of phaseoloidin, and traces of entadamide A glucone not exceeding a weight content of 0.2 mg/g.

5. The process for producing an extract of seeds of the genus Entada enriched in entadamide A according to any one of claims 1 to 4, wherein the phaseoloidin/entadamide A weight ratio is between 15 and 25, and the entadamide A glucone/entadamide A weight ratio is less than 0.5.

6. The process for producing an extract of seeds of the genus Entada enriched in entadamide A according to any one of claims 1 to 5, wherein the inhibitory agent of the activity of endogenous enzymes of step (iii/a) is an organic solvent miscible with water, advantageously glucose-added water or an alcohol chosen from ethanol, methanol, propanol and its isomers, butanol and its isomers, pentanol, hexanol, and mixtures thereof.

7. The process for producing an extract of seeds of the genus Entada enriched in entadamide A according to claim 6, wherein said agent is ethanol.

8. The process for producing an extract of seeds of the genus Entada enriched in entadamide A according to any one of claims 1 to 7, wherein the cosmetically acceptable solvent of step (iv) is a glycolic solvent, advantageously chosen from 1,3-propanediol, 1,2-propanediol, methylpropanediol, phenoxypropanediol, 1,2-butanediol, 1,3-butanediol, 1,4-butanediol, 2,3-butanediol, 1,2-hexanediol, 1,2-dihydroxyethane, diethylene glycol, dipropylene glycol, triethylene glycol, tripropylene glycol, diethoxy diglycol, pentylene glycol, hexylene glycol, 1,2-octanediol, glycerin, and mixtures thereof.

9. The process for producing an extract of seeds of the genus Entada enriched in entadamide A according to claim 8, wherein said solvent is 1,3-propanediol.

10. A seed extract of the genus Entada enriched in entadamide A obtainable by the process according to any one of claims 1 to 9.

11. A seed extract of the genus Entada enriched in entadamide A according to claim 10, which comprises a weight content of between 1 and 5 mg/g of entadamide A, a weight content of between 9 and 85 mg/g of phaseoloidin, and traces of entadamide A glucone not exceeding a weight content of 0.2 mg/g.

12. A seed extract of the genus Entada enriched in entadamide A according to any one of claims 10 and 11 for use for preventing or combating the effects of solar radiation harmful to the skin, in particular ultraviolet-B radiation.

13. A seed extract of the genus Entada enriched in entadamide A according to any one of claims 10 and 11 for use for preventing or combating skin signs resulting from atmospheric pollution, from contact with chemical xenobiotics or smoky atmospheres.

14. Non-therapeutic cosmetic use of a seed extract of the genus Entada enriched in entadamide A according to any one of claims 10 and 11, as an agent useful for maintaining the homeostasis of the microbiome present at the skin surface.

15. A seed extract of the genus Entada enriched in entadamide A according to any one of claims 10 and 11, for dermocosmetic use in the treatment of acne, seborrhea, rosacea or atopic dermatitis.

16. A composition for cosmetic or dermocosmetic use, which comprises, as main active ingredient, a seed extract of the genus Entada enriched in entadamide A according to any one of claims 10 and 11, in combination with one or more additives physiologically compatible with skin or appendages, and wherein the amount of said extract is between 1% and 10% by weight relative to the total weight of the composition.
